(19) Europäisches Patentamt
European Patent Office
Office européen des brevets

(11) Veröffentlichungsnummer: **0 182 746**
**A2**

(12) # EUROPÄISCHE PATENTANMELDUNG

(21) Anmeldenummer: **85810529.9**

(22) Anmeldetag: **11.11.85**

(51) Int. Cl.⁴: **C 07 D 231/06**, A 01 N 43/56

(30) Priorität: **16.11.84 CH 5489/84**
**22.10.85 CH 4551/85**

(43) Veröffentlichungstag der Anmeldung: **28.05.86**
**Patentblatt 86/22**

(84) Benannte Vertragsstaaten: **BE CH DE FR GB IT LI NL**

(71) Anmelder: **CIBA-GEIGY AG, Klybeckstrasse 141,**
**CH-4002 Basel (CH)**

(72) Erfinder: **Farooq, Saleem, Dr., Kirchackerstrasse 27,**
**CH-4422 Arlsdorf (CH)**

(54) **Pyrazolinderivate.**

(57) Neue 1-Carbamoyl-3-phenyl-4-benzyl-$\Delta^2$-pyrazoline der Formel

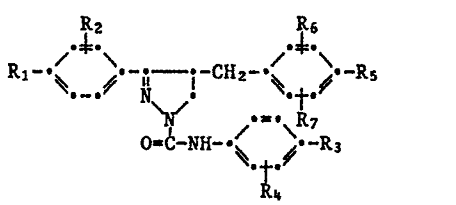

(I),

worin

$R_1$ und $R_2$ unabhängig voneinander Wasserstoff, Halogen, Trifluormethyl, $C_1$-$C_4$-Halogenalkoxy mit 1 bis 7 Halogenatomen oder $C_1$-$C_4$-Halogenalkylthio mit 1 bis 7 Halogenatomen;

$R_3$ Wasserstoff, Halogen, $C_1$-$C_4$-Alkyl, $C_1$-$C_4$-Halogenalkyl mit 1 bis 7 Halogenatomen, $C_1$-$C_4$-Alkoxy, $C_1$-$C_4$-Halogenalkoxy mit 1 bis 7 Halogenatomen oder Alkoxycarbonyl mit 1 bis 4 C-Atomen im Alkylteil;

$R_4$ Wasserstoff, Halogen, $C_1$-$C_4$-Alkyl oder Trifluormethyl;

$R_5$ Wasserstoff, Halogen, $C_1$-$C_4$-Alkyl, Trifluormethyl oder $C_1$-$C_4$-Alkoxy, und

$R_6$ und $R_7$ unabhängig voneinander Wasserstoff, Halogen oder Trifluormethyl oder $R_6$ bzw. $R_7$ zusammen mit $R_5$ Methylendioxy

bedeuten; Verfahren zur Herstellung dieser Verbindungen sowie diese enthaltende Mittel zur Bekämpfung von Insekten und Vertretern der Ordnung Akarina, speziell von pflanzenschädigenden Frass-Insekten und Bodeninsekten.

EP 0 182 746 A2

0182746

CIBA-GEIGY AG
Basel (Schweiz)

5-15161/1+2

# Pyrazolinderivate

Die vorliegende Erfindung betrifft neue 1-Carbamoyl-3-phenyl-4-benzyl-$\Delta^2$-pyrazoline, Verfahren zu ihrer Herstellung und ihre Verwendung in der Schädlingsbekämpfung.

Die erfindungsgemässen Verbindungen haben die Formel I

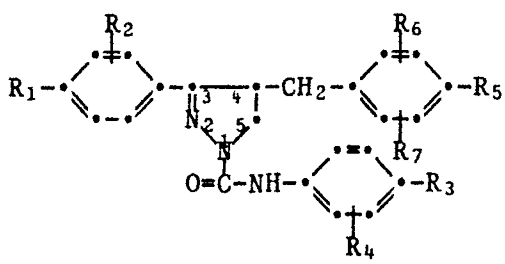

(I) ,

worin

$R_1$ und $R_2$ unabhängig voneinander Wasserstoff, Halogen, Trifluormethyl, $C_1$-$C_4$-Halogenalkoxy mit 1 bis 7 Halogenatomen oder $C_1$-$C_4$-Halogenalkylthio mit 1 bis 7 Halogenatomen;

$R_3$ Wasserstoff, Halogen, $C_1$-$C_4$-Alkyl, $C_1$-$C_4$-Halogenalkyl mit 1 bis 7 Halogenatomen, $C_1$-$C_4$-Alkoxy, $C_1$-$C_4$-Halogenalkoxy mit 1 bis 7 Halogenatomen oder Alkoxycarbonyl mit 1 bis 4 C-Atomen im Alkylteil;

$R_4$ Wasserstoff, Halogen, $C_1$-$C_4$-Alkyl oder Trifluormethyl;

$R_5$ Wasserstoff, Halogen, $C_1$-$C_4$-Alkyl, Trifluormethyl oder $C_1$-$C_4$-Alkoxy; und

$R_6$ und $R_7$ unabhängig voneinander Wasserstoff, Halogen oder Trifluormethyl oder $R_6$ bzw. $R_7$ zusammen mit $R_5$ Methylendioxy

bedeuten.

Hervorzuheben sind ferner diejenigen Verbindung der Formel I, worin $R_2$, $R_4$, $R_6$ und $R_7$ Wasserstoff bedeuten.

Bevorzugte Verbindungen der Formel I sind dadurch gekennzeichnet, dass

$R_1$ Wasserstoff, Fluor, Chlor oder $C_1$-$C_3$-Halogenalkoxy mit 1 bis 7 Fluor- oder Chloratomen;

$R_2$ Wasserstoff;

$R_3$ Wasserstoff, Fluor, Chlor, $C_1$-$C_3$-Alkoxy, $C_1$-$C_3$-Halogenalkoxy mit 1 bis 7 Fluor-, Chlor- oder Bromatomen oder i-Propoxycarbonyl;

$R_4$ Wasserstoff, Methyl oder Aethyl;

$R_5$ Wasserstoff, Fluor, Chlor, Brom, $C_1$-$C_3$-Alkyl, Trifluormethyl oder $C_1$-$C_3$-Alkoxy;

$R_6$ Wasserstoff, in 2- oder 3-Stellung befindliches Fluor oder Chlor oder zusammen mit $R_5$ eine 3,4-Methylendioxygruppe; und

$R_7$ Wasserstoff oder 6-Chlor

bedeuten.

Weiterhin bevorzugt sind solche Verbindungen der Formel I, worin

$R_1$ Wasserstoff, Fluor, Chlor oder Methyl;

$R_2$ Wasserstoff;

$R_3$ Fluor, Chlor oder Trifluormethoxy, Difluorbrommethoxy oder i-Propoxycarbonyl;

$R_4$ Wasserstoff oder 3-Methyl;

$R_5$ Wasserstoff, Fluor, Chlor, Brom oder Methyl;

$R_6$ Wasserstoff oder 3-Chlor; und

$R_7$ Wasserstoff

bedeuten, sowie solche Verbindungen der Formel I, worin

$R_1$ Wasserstoff, Fluor oder Chlor;

$R_2$, $R_4$, $R_6$ und $R_7$ Wasserstoff;

$R_3$ Chlor oder Trifluormethoxy; und

$R_5$ Fluor oder Chlor

bedeuten.

Unter dem Begriff Alkyl selbst oder als Bestandteil eines anderen Substituenten sind geradkettige oder verzweigte und je nach Zahl der angegebenen Kohlenstoffatome im Rahmen der vorliegenden Erfindung beispielsweise folgende Gruppen zu verstehen: Methyl, Ethyl, Propyl, Butyl, sowie ihre Isomeren, wie Isopropyl, Isobutyl, tert.-Butyl, sek.-Butyl.

Unter dem Begriff Halogenalkyl im Rahmen der vorliegenden Erfindung sind Alkylreste, wie Methyl, Aethyl, n-Propyl und Isopropyl, Butyl usw. zu verstehen, die mit 1 bis 7 unterschiedlichen oder gleichartigen Halogenatomen substituiert sind, wobei es sich um perhalogenierte Alkylreste aber auch um solche handeln kann, deren Wasserstoffatome nur teilweise mit Halogen substituiert sind.

Unter dem Begriff Halogen im Rahmen der vorliegenden Erfindung ist Fluor, Chlor und Brom zu verstehen, vorzugsweise Fluor und Chlor.

Die Verbindungen der Formel I können analog an sich bekannten Verfahren hergestellt werden (vgl. z.B. die deutschen Offenlegungsschriften Nr. 2.529.689, 2.700.258 und die europäische Patentanmeldung Nr. 0.004.733.)

So kann man z.B. eine Verbindung der Formel I erhalten, indem man eine Verbindung der Formel II

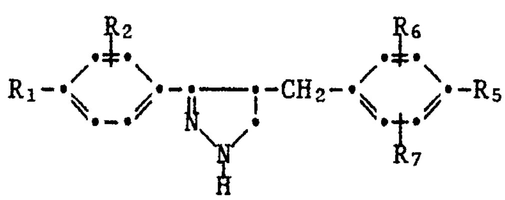

(II)

mit einer Verbindung der Formel III

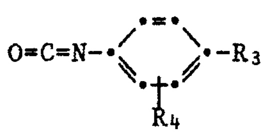

(III)

umsetzt, wobei in den Formeln II und III die Reste $R_1$ bis $R_7$ die unter Formel I vorstehend angegebenen Bedeutungen haben.

Das erwähnte Verfahren kann vorzugsweise unter normalem Druck und in Gegenwart eines gegenüber den Reaktionsteilnehmern inerten organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Das Verfahren lässt sich im allgemeinen gut bei einer Temperatur von -10 bis 100°C, vorzugsweise zwischen 10 und 30°C, z.B. bei Raumtemperatur, gegebenenfalls in Gegenwart einer geeigneten aprotischen organischen Base, z.B. Triäthylamin, N-Methylmorpholin oder Pyridin, durchführen.

Die Ausgangsstoffe der Formeln II und III sind bekannt und können analog bekannter Verfahren hergestellt werden. Die 3-Phenyl-4-benzyl-$\Delta^2$-pyrazoline der Formel II kann man z.B. erhalten, indem man ein entsprechendes ß-Phenylpropiophenon der Formel IV

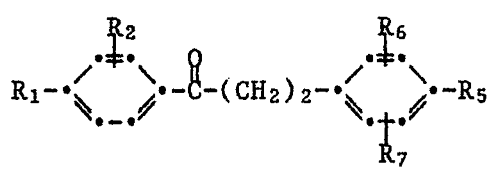

(IV)

bei erhöhter Temperatur in Gegenwart eines Katalysators mit Formaldehyd umsetzt und das erhaltene 1-Phenyl-2-benzyl-prop-2-en-1-on der Formel V

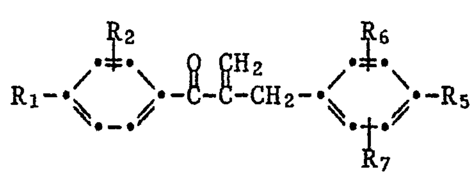

(V)

durch Kondensation mit Hydrazin cyclisiert (vgl. die vorstehend genannten Schrifttumstellen).

Das Propiophenon der Formel IV ist durch Hydrierung eines entsprechenden Benzylidenacetophenons der Formel VI

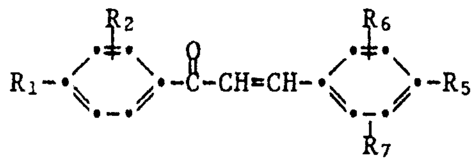

(VI)

nach allgemein üblicher Arbeitsweise zugänglich. In den obigen Formeln IV bis VI haben $R_1$, $R_2$, $R_5$, $R_6$ und $R_7$ die vorstehend unter Formel I angegebenen Bedeutungen.

Aus den deutschen Offenlegungsschriften Nr. 2 529 689 und 2 700 258 sowie aus der europäischen Patentanmeldung 0 004 733 sind bereits substituierte 1-Carbamoyl-3-phenyl-$\Delta^2$-pyrazoline mit insektizider Wirkung bekannt. Die vorliegenden Verbindungen der Formel I unterscheiden sich demgegenüber strukturell vor allem durch das Vorliegen einer Benzyl-Gruppe anstatt eines Phenyl-Restes in 4-Stellung am $\Delta^2$-Pyrazolinring.

Ueberraschenderweise wurde gefunden, dass die vorliegenden Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte larvizide Wirkung gegen Insekten, insbesondere Larven und Nymphen von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen. Die erfindungsgemässen Verbindungen eignen sich weiterhin zur Bekämpfung von Bodeninsekten (z.B. Aulacophora femoralis, Chortophila brassicae, Diabrotica balteata, Pachnoda savigni und Scotia ypsilon). Auch können die Verbindungen der Formel I mit ausgezeichnetem Erfolg gegen pflanzenschädigende Zikaden, speziell in Reis-Kulturen, eingesetzt werden, wobei sie sowohl systemische als auch Kontakt-Wirkung zeigen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Durch den Zusatz von Verbindungen der Formel I kann die insektizide Wirkung des bekannten Insektizids Dimethyl-(Z)-1-methyl-2-methylcarbamoyl-vinylphosphat gesteigert bzw. aktiviert werden.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie

N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die erfindungsgemässen pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

a) Herstellung von 4,4'-Difluor-ß-phenylpropiophenon

Es werden 59,9 g 4,4'-Difluorbenzylidenacetophenon gelöst in 600 ml Tetrahydrofuran in Anwesenheit von 6 g Raney-Nickel hydriert. Das vom Katalysator befreite Reaktionsgemisch wird am Rotationsverdampfer eingedampft. Man erhält die Titelverbindung der Formel

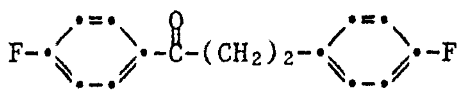

als Rohprodukt mit einem Brechungsindex $n_{20}^{D}$ = 1,5478.

b) Herstellung von 3-(4-Fluorphenyl)-4-(4-fluorbenzyl)-$\Delta^2$-pyrazolin

Es werden 59,4 g des gemäss a) hergestellten 4,4'-Difluor-ß-phenylpropiophenons zusammen mit 39,2 g Dimethylamin-Hydrochlorid und 14,4 g Paraformaldehyd über Nacht am Rückfluss erhitzt. Dann wird das Reaktionsgemisch eingedampft, der Rückstand mit 300 ml Wasser versetzt und dreimal mit je 200 ml Aether extrahiert. Die vereinigten Aether-Extrakte wurden zweimal mit Wasser und zweimal mit gesättigter Kochsalzlösung gewaschen, über Natriumsulfat getrocknet, filtriert und eingeengt. Man erhält das 1-(4-Fluorphenyl)-2-(4-fluorbenzyl)-prop-2-en-1-on der Formel

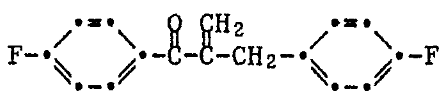

als Rohprodukt, das sofort weiter umgesetzt wird. Das Rohprodukt wird in 140 ml Aethanol gelöst, und zu dieser Lösung werden 18,8 g Hydrazin-Hydrat gelöst in 70 ml Methanol zugetropft. Nach Beendigung der Zugabe wird das Reaktionsgemisch während drei Stunden am Rückfluss erhitzt, abgekühlt und dann am Rotationsverdampfer

eingeengt. Der erhaltene Rückstand wird in Aether aufgenommen, zweimal mit Wasser und zweimal mit gesättigter Kochsalzlösung gewaschen, über $Na_2SO_4$ getrocknet, filtriert und eingedampft.

Man erhält auf diese Weise die Titelverbindung der Formel

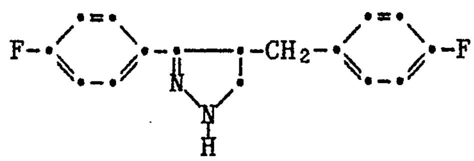

.

c) Herstellung von 1-(4-Trifluormethoxyphenyl-carbamoyl)-3-(4-fluorphenyl)-4-(4-fluorbenzyl)-$\Delta^2$-pyrazolin

Es werden 20 g des gemäss b) erhaltenen 3-(4-Fluorphenyl)-4-(4-fluorbenzyl)-$\Delta^2$-pyrazolins in 50 ml Aether vorgelegt. In diese Lösung werden 14,9 g 4-Trifluormethoxyphenylisocyanat unter Eiskühlung eingetropft. Nach einer Stunde bei Raumtemperatur wird das ausgefallene Produkt abfiltriert, mit Aether gewaschen und getrocknet. Die so erhaltene Titelverbindung der Formel

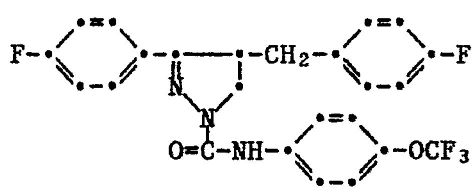

hat einen Schmelzpunkt von 98 - 100°C (Verbindung Nr. 1).

Analog den oben beschriebenen Arbeitsweisen werden auch die folgenden Verbindungen der Formel I hergestellt:

| Verbindung Nr. | $R_1$ | $R_2$ | $R_3$ | $R_4$ | $R_5$ | $R_6$ | $R_7$ | physikalische Daten |
|---|---|---|---|---|---|---|---|---|
| 2 | H | H | H | H | 3,4-O-$CH_2$-O- | | H | Smp.107-109°C |
| 3 | H | H | H | 3-$CH_3$ | 3,4-O-$CH_2$-O- | | H | Smp.121-123°C |
| 4 | H | H | -CO-O-CH($CH_3$)$_2$ | H | 3,4-O-$CH_2$-O- | | H | zähflüssige Masse |
| 5 | H | H | H | H | Cl | H | H | Smp.142-144°C |
| 6 | H | H | H | 3-$CH_3$ | Cl | H | H | Smp.134-135°C |
| 7 | H | H | -O-$CF_3$ | H | Cl | H | H | Smp.114-116°C |
| 8 | H | H | -O-$CBrF_2$ | H | Cl | H | H | Smp.109-111°C |
| 9 | H | H | H | H | Br | H | H | Smp.150-152°C |
| 10 | H | H | H | 3-$CH_3$ | Br | H | H | Smp.132-135°C |
| 11 | H | H | -O-$CF_3$ | H | Br | H | H | Smp.128-129°C |
| 12 | H | H | H | H | F | H | H | Smp.128-130°C |
| 13 | H | H | -CO-O-CH($CH_3$)$_2$ | H | Cl | H | H | Smp. 68-70°C |
| 14 | H | H | H | 3-$CH_3$ | F | H | H | Smp.129-131°C |

| Verbindung Nr. | $R_1$ | $R_2$ | $R_3$ | $R_4$ | $R_5$ | $R_6$ | $R_7$ | physikalische Daten |
|---|---|---|---|---|---|---|---|---|
| 15 | H | H | $-O-CF_3$ | H | F | H | H | Smp.107-108°C |
| 16 | H | H | H | H | $-CH_3$ | H | H | Smp.128-130°C |
| 17 | H | H | $-CO-O-CH(CH_3)_2$ | H | $-CH_3$ | H | H | Smp. 67-69°C |
| 18 | H | H | H | $3-CH_3$ | $-CH_3$ | H | H | Smp.126-128°C |
| 19 | H | H | $-O-CF_3$ | H | $-CH_3$ | H | H | Smp.134-136°C |
| 20 | H | H | H | H | Cl | 3-Cl | H | Smp.129-131°C |
| 21 | H | H | H | $3-CH_3$ | Cl | 3-Cl | H | Smp.134-136°C |
| 22 | H | H | $-O-CF_3$ | H | Cl | 3-Cl | H | Smp.118-120°C |
| 23 | H | H | H | H | H | 6-Cl | 2-Cl | Smp.188-190°C |
| 24 | H | H | H | $3-CH_3$ | H | 6-Cl | 2-Cl | Smp.178-180°C |
| 25 | H | H | $-O-CF_3$ | H | H | 6-Cl | 2-Cl | Smp.180-182°C |
| 26 | Cl | H | H | H | Cl | H | H | Smp.133-135°C |
| 27 | F | H | H | H | F | H | H | Smp.124-126°C |
| 28 | F | H | H | $3-CH_3$ | F | H | H | Smp. 96-98°C |

| Verbin-dung Nr. | $R_1$ | $R_2$ | $R_3$ | $R_4$ | $R_5$ | $R_6$ | $R_7$ | physikalische Daten |
|---|---|---|---|---|---|---|---|---|
| 29 | $-CH_3$ | H | H | H | $-CH_3$ | H | H | Smp. 151-153°C |
| 30 | $-CH_3$ | H | H | $3-CH_3$ | $-CH_3$ | H | H | Smp. 130-132°C |
| 31 | $-CH_3$ | H | $-O-CF_3$ | H | $-CH_3$ | H | H | Smp. 108-109°C |
| 32 | $-CH_3$ | H | H | H | Cl | 3-Cl | H | Smp. 160-162°C |
| 33 | $-CH_3$ | H | H | $3-CH_3$ | Cl | 3-Cl | H | zähflüssige Masse |
| 34 | $-CH_3$ | H | $-O-CF_3$ | H | Cl | 3-Cl | H | Smp. 143-145°C |
| 35 | Cl | H | H | H | H | H | H | Smp. 70-72°C |
| 36 | Cl | H | H | $3-CH_3$ | H | H | H | Smp. 104-106°C |
| 37 | Cl | H | $-O-CF_3$ | H | H | H | H | Smp. 133-135°C |
| 38 | Cl | H | H | H | F | H | H | Smp. 126-128°C |
| 39 | Cl | H | H | $3-CH_3$ | F | H | H | Smp. 130-132°C |
| 40 | Cl | H | $-O-CF_3$ | H | F | H | H | Smp. 141-142°C |

| Verbindung Nr. | $R_1$ | $R_2$ | $R_3$ | $R_4$ | $R_5$ | $R_6$ | $R_7$ | physikalische Daten |
|---|---|---|---|---|---|---|---|---|
| 41 | Cl | H | F | H | F | H | H | Smp. 149-152°C |
| 42 | Cl | H | H | H | H | 2-Cl | 6-Cl | Smp. 144-146°C |
| 43 | Cl | H | H | 3-$CH_3$ | H | 2-Cl | 6-Cl | Smp. 174-176°C |
| 44 | Cl | H | -O-$CF_3$ | H | H | 2-Cl | 6-Cl | Smp. 192-193°C |
| 45 | H | 2-Cl | H | H | Cl | H | H | zähflüssige Masse |
| 46 | H | 2-Cl | H | 3-$CH_3$ | Cl | H | H | Smp. 182-184°C |
| 47 | H | 2-Cl | -O-$CF_3$ | H | Cl | H | H | zähflüssige Masse |
| 48 | H | 2-Cl | F | H | Cl | H | H | zähflüssige Masse |
| 49 | H | 2-Cl | H | H | -$CH_3$ | H | H | Smp. 141-143°C |
| 50 | H | 2-Cl | -O-$CF_3$ | H | -$CH_3$ | H | H | $n_{20}^{D}$ = 1,5695 |
| 51 | F | H | Cl | H | F | H | H | Smp. 140-142°C |
| 52 | F | H | F | H | F | H | H | Smp. 127-130°C |

| Verbindung Nr. | $R_1$ | $R_2$ | $R_3$ | $R_4$ | $R_5$ | $R_6$ | $R_7$ | physikalische Daten |
|---|---|---|---|---|---|---|---|---|
| 53 | Cl | H | $-O-CF_3$ | H | Cl | H | H | Smp. 139-140°C |
| 54 | Cl | H | Cl | H | Cl | H | H | Smp. 64-68°C |
| 55 | Cl | H | F | H | Cl | H | H | Smp. 156-158°C |
| 56 | F | H | $-O-CF_3$ | H | H | H | H | Smp. 106-107°C |
| 57 | F | H | F | H | H | H | H | Smp. 111-112°C |
| 58 | F | H | Cl | H | H | H | H | zähflüssige Masse |
| 59 | F | H | H | H | Cl | H | H | Smp. 123-125°C |
| 60 | F | H | H | $3-CH_3$ | Cl | H | H | Smp. 130-134°C |
| 61 | F | H | $-O-CF_3$ | H | Cl | H | H | Smp. 148-150°C |
| 62 | F | H | H | $3-CF_3$ | Cl | H | H | zähflüssige Masse |
| 63 | F | H | Cl | H | Cl | H | H | Smp. 110-112°C |
| 64 | F | H | F | H | Cl | H | H | Smp. 125-127°C |

| Verbindung Nr. | $R_1$ | $R_2$ | $R_3$ | $R_4$ | $R_5$ | $R_6$ | $R_7$ | physikalische Daten |
|---|---|---|---|---|---|---|---|---|
| 65 | Cl | H | $-CO-O-CH(CH_3)_2$ | H | Cl | H | H | Smp. 86-88°C |
| 66 | Cl | H | $-OCF_2CHF_2$ | H | Cl | H | H | Smp. 124-126°C |
| 67 | F | H | $-OCF_2CHF_2$ | H | F | H | H | Smp. 134-136°C |
| 68 | F | H | $-CO-O-CH(CH_3)_2$ | H | F | H | H | Smp. 65-68°C |
| 69 | Cl | H | $-CO-O-CH(CH_3)_2$ | H | F | H | H | Smp. 74-76°C |
| 70 | Cl | H | $-OCF_2CHF_2$ | H | F | H | H | Smp. 160-162°C |
| 71 | F | H | $-CO-O-CH(CH_3)_2$ | H | Cl | H | H | Smp. 70-72°C |
| 72 | F | H | $-OCF_2CHF_2$ | H | Cl | H | H | Smp. 145-147°C |
| 73 | F | H | H | $3-CF_3$ | F | H | H | Smp. 110-112°C |
| 74 | F | H | $-O-CH_3$ | H | F | H | H | Smp. 106-111°C |
| 75 | F | H | $-CH_3$ | H | F | H | H | Smp. 146-148°C |
| 76 | F | H | H | 3-Cl | F | H | H | Smp. 138-140°C |
| 77 | F | H | $-CF_3$ | H | F | H | H | Smp. 189-191°C |

| Verbindung Nr. | $R_1$ | $R_2$ | $R_3$ | $R_4$ | $R_5$ | $R_6$ | $R_7$ | physikalische Daten |
|---|---|---|---|---|---|---|---|---|
| 78 | H | H | $-CO-O-CH(CH_3)_2$ | H | 3,4-$O-CH_2-O$- | | H | zähflüssige Masse |
| 79 | $-O-CHF_2$ | H | $-O-CF_3$ | H | F | H | H | Smp. 99-100°C |
| 80 | $-O-CHF_2$ | H | F | H | F | H | H | Smp. 147-148°C |
| 81 | $-O-CHF_2$ | H | Cl | H | F | H | H | Smp. 134-136°C |
| 82 | $-O-CHF_2$ | H | H | H | F | H | H | Smp. 109-111°C |
| 83 | F | H | Cl | 3-Cl | F | H | H | Smp. 195-196°C |
| 84 | F | H | H | 3-F | F | H | H | Smp. 121-122°C |
| 85 | F | H | H | 2-$CF_3$ | F | H | H | Smp. 107-108°C |
| 86 | F | H | Br | H | F | H | H | Smp. 152-154°C |
| 87 | F | H | H | 2-F | F | H | H | Smp. 140-142°C |

Wie vorstehend ausgeführt sind auch folgende Verbindungen der Formel I erhältlich:

| Verbindung Nr. | $R_1$ | $R_2$ | $R_3$ | $R_4$ | $R_5$ | $R_6$ | $R_7$ |
|---|---|---|---|---|---|---|---|
| 88 | Cl | H | Cl | H | F | H | H |
| 89 | F | H | Cl | H | $-CF_3$ | H | H |
| 90 | F | H | $-O-CF_3$ | H | $-CF_3$ | H | H |
| 91 | F | H | $-CO-O-CH(CH_3)_2$ | H | $-CF_3$ | H | H |
| 92 | $-O-CHF_2$ | H | Cl | H | Cl | H | H |
| 93 | $-O-CHF_2$ | H | Cl | H | $CF_3$ | H | H |
| 94 | $-O-CHF_2$ | H | $-O-CF_3$ | H | Cl | H | H |
| 95 | $-O-CHF_2$ | H | $-O-CF_3$ | H | $CF_3$ | H | H |
| 96 | $-O-CHF_2$ | H | $-CO-O-CH(CH_3)_2$ | H | F | H | H |
| 97 | $-O-CHF_2$ | H | $-CO-O-CH(CH_3)_2$ | H | Cl | H | H |
| 98 | $-O-CHF_2$ | H | $-CO-O-CH(CH_3)_2$ | H | $CF_3$ | H | H |

Beispiel 2:

Formulierungen für flüssige Wirkstoffe der Formel I gemäss Beispiel 1 oder Kombinationen dieser Wirkstoffe mit andern Insektiziden oder Akariziden (% = Gewichtsprozent)

| 1. Emulsions-Konzentrate | a) | b) | c) |
|---|---|---|---|
| Wirkstoff resp. Wirkstoffkombination | 25 % | 40 % | 50 % |
| Ca Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexan | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

2. <u>Lösungen</u>

| | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

3. <u>Granulate</u>

| | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff bzw. die Wirkstoffkombination wird in Methylenchlorid gelöst, auf den Träger aufgesprüht und das Lösungsmittel anschliessend im Vakuum abgedampft.

4. <u>Stäubemittel</u>

| | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff bzw. der Wirkstoffkombination erhält man gebrauchsfertige Stäubemittel.

Formulierungen für feste Wirkstoffe der Formel I gemäss Beispiel 1 resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Wirkung gegen Musca domestica:
Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoff-konzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 4: Wirkung gegen Aëdes aegypti:
Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

Beispiel 5: Insektizide Frassgift-Wirkung:

Ca. 25 cm hohe eingetopfte Baumwollpflanzen werden mit wässrigen Wirkstoffemulsionen besprüht, die den Wirkstoff jeweils in Konzentrationen von 100, 200 und 400 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und 60 % relativer Luftfeuchtigkeit durchgeführt. Nach 120 Stunden wird die %-Mortalität der Test-Insekten bestimmt.

Erfindungsgemässe Verbindungen zeigen in diesem Test bei den nachstehend aufgeführten Wirkstoff-Konzentrationen eine Wirkung von 80-100% (Mortalität):

| Verbindung Nr. | Wirkstoffkonzentration | |
|---|---|---|
| | Spodoptera | Heliothis |
| 13 | 400 ppm | - |
| 15 | 400 ppm | 400 ppm |
| 31 | 400 ppm | - |
| 37 | 400 ppm | 400 ppm |
| 40 | 400 ppm | 200 ppm |
| 51 | - | 100 ppm |
| 54 | - | 400 ppm |
| 56 | 400 ppm | 400 ppm |

Beispiel 6: Wirkung gegen Anthonomus grandis (Adulte)

Eingetopfte Baumwollpflanzen im Keimblattstadium werden mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten ungesexten Käfern (Anthonomus grandis) besiedelt. Plastikzylinder, deren obere Oeffnungen mit Gaze abgedeckt sind, werden dann über die behandelten, mit den Testtieren besiedelten Pflanzen

gestülpt, um ein Abwandern der Käfer zu verhindern. Die so behandelten Pflanzen werden bei 25°C und etwa 60 % relativer Luftfeuchtigkeit gehalten. Die Auswertung auf Frassgift-Wirkung erfolgt nach 1, 2, 3 und 6 Tagen unter Zugrundelegung der prozentualen Mortalität der eingesetzten Testtiere (% Rückenlage).

Die überlebenden Käfer werden für 3 Tage zur Eiablage auf Anthonomus-Diät-Pellets transferiert. Dann werden die Käfer entfernt und die mit den Eigelegen versehenen Pellets während 7 Tagen bei 28°C inkubiert. Die Auswertung auf den die Reproduktionsfähigkeit beeinflussenden Effekt erfolgt anhand der Verminderung der Anzahl abgelegter Eier, der daraus geschlüpften Larven und der Anzahl toter Larven im Vergleich zu unbehandelten Kontrollen.

Bei einer Wirkstoff-Konzentration von 400 ppm wird hinsichtlich der Frassgift-Wirkung mit den Verbindungen Nr. 1, 4, 13 und 15 und hinsichtlich der Reproduktions-Beeinflussung mit den Verbindungen Nr. 7, 8, 18, 19, 20 und 22 gemäss Beispiel 1 eine Wirkung von 80-100 % (Mortalität) gegenüber unbehandelten Kontrollen erzielt.

Beispiel 7: Wirkung gegen Bodeninsekten (Diabrotica balteata)
Es werden 5 Maiskeimlinge von 1 -3 cm sowie eine Filterpapier-Rondelle in eine wässrige Wirkstofflösung enthaltend etwa 4 Vol.-% Aceton eingetaucht. Die eingetauchte Filterpapier-Rondelle wird auf den Boden eines Kunststoff-Bechers (Inhalt 200 ml) gelegt und darauf wird eine trockene Filterpapier-Rondelle sowie die Maiskeimlinge und 10 Larven von Diabrotica balteata im 2. oder 3. Larvalstadium gegeben. Der Ansatz wird bei ca. 24°C und 40 - 60 % relativer Luftfeuchtigkeit und Tageslicht gehalten. Die Bonitur erfolgt nach 6 Tagen gegenüber unbehandelten Kontrollansätzen.

Erfindungsgemässe Verbindungen zeigen in diesem Test bei den nachstehend aufgeführten Wirkstoff-Konzentrationen eine Wirkung von 80-100 % (Mortalität):

| Verbindung Nr | Wirkstoffkonzentration |
|---|---|
| 1 und 53 | 3 ppm |
| 11, 15 und 40 | 12,5 ppm |
| 7, 51, 56 und 61 | 50 ppm |
| 4, 37, 54 und 63 | 100 ppm |
| 58 | 200 ppm |
| 1 und 6 | 400 ppm |

Beispiel 8: Wirkung gegen Laodelphax striatellus und Nilaparvate lugens (Nymphen)

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein Glaszylinder gestülpft und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Erfindungsgemässe Verbindungen der Formel I gemäss Beispiel 1 zeigen in diesem Test gute Wirksamkeit.

Beispiel 9: Wirkung gegen Laodelphax striatellus und Nilaparvata lugens (ovizid)

Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einem Drehteller mit jeweils 100 ml einer acetonischen Lösung enthaltend 400 ppm Wirkstoff besprüht. Nach dem Antrocknen des Spritzbelages wird jede Pflanze mit 3 adulten Weibchen besiedelt. Um die Tiere am Entweichen zu hindern, wird über jede besiedelte Pflanze ein Glaszylinder gestülpt und dieser mit einem Gänze-Deckel abgedeckt. Die Weibchen bleiben 4 Tage zur Eiablage auf der behandelten Pflanze und werden dann entfernt.

Etwa 8 Tage nach Besiedlung schlüpfen die jungen Zikaden aus den Eiern und es erfolgt die Auszählung. Aus dem Vergleich der Anzahl geschlüpfter Larven auf den behandelten Pflanzen zu den auf unbehandelten Kontrollpflanzen geschlüpften Tieren wird die prozentuale Mortalität berechnet.

Verbindungen der Formel I gemäss Beispiel 1 zeigen in obigem Test gute ovizide Wirkung.

Patentansprüche:

1. Verbindung der Formel I,

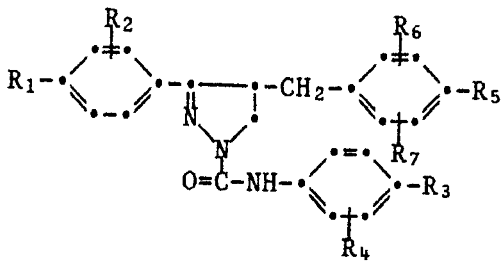 (I) ,

worin

$R_1$ und $R_2$ unabhängig voneinander Wasserstoff, Halogen, Trifluormethyl, $C_1$-$C_4$-Halogenalkoxy mit 1 bis 7 Halogenatomen oder $C_1$-$C_4$-Halogenalkylthio mit 1 bis 7 Halogenatomen;

$R_3$ Wasserstoff, Halogen, $C_1$-$C_4$-Alkyl, $C_1$-$C_4$-Halogenalkyl mit 1 bis 7 Halogenatomen, $C_1$-$C_4$-Alkoxy, $C_1$-$C_4$-Halogenalkoxy mit 1 bis 7 Halogenatomen oder Alkoxycarbonyl mit 1 bis 4 C-Atomen im Alkylteil;

$R_4$ Wasserstoff, Halogen, $C_1$-$C_4$-Alkyl oder Trifluormethyl;

$R_5$ Wasserstoff, Halogen, $C_1$-$C_4$-Alkyl, Trifluormethyl oder $C_1$-$C_4$-Alkoxy; und

$R_6$ und $R_7$ unabhängig voneinander Wasserstoff, Halogen oder Trifluormethyl oder $R_6$ bzw. $R_7$ zusammen mit $R_5$ Methylendioxy bedeuten.

2. Verbindung gemäss Anspruch 1, dadurch gekennzeichnet, dass $R_2$, $R_4$, $R_6$ und $R_7$ Wasserstoff bedeuten.

3. Verbindung gemäss Anspruch 1, dadurch gekennzeichnet, dass

$R_1$ Wasserstoff, Fluor, Chlor oder $C_1$-$C_3$-Halogenalkoxy mit 1 bis 7 Fluor- oder Chloratomen;

$R_2$ Wasserstoff;

$R_3$ Wasserstoff, Fluor, Chlor, $C_1$-$C_3$-Alkoxy, $C_1$-$C_3$-Halogenalkoxy mit 1 bis 7 Fluor-, Chlor- oder Bromatomen oder i-Propoxacarbonyl;

$R_4$ Wasserstoff, Methyl oder Aethyl;

$R_5$ Wasserstoff, Fluor, Chlor, Brom, $C_1$-$C_3$-Alkyl, Trifluormethyl oder $C_1$-$C_3$-Alkoxy;

$R_6$ Wasserstoff, in 2- oder 3-Stellung befindliches Fluor oder Chlor oder zusammen mit $R_5$ eine 3,4-Methylendioxygruppe, und

$R_7$ Wasserstoff oder 6-Chlor

bedeuten.

4. Verbindung gemäss Anspruch 3, dadurch gekennzeichnet, dass

$R_1$ Wasserstoff, Fluor, Chlor oder Methyl;

$R_2$ Wasserstoff;

$R_3$ Fluor, Chlor, oder Trifluormethoxy;

$R_4$ Wasserstoff oder 3-Methyl;

$R_5$ Wasserstoff, Fluor, Chlor, Brom oder Methyl;

$R_6$ Wasserstoff oder 3-Chlor; und

$R_7$ Wasserstoff

bedeuten.

5. Verbindung gemäss A.2, dadurch gekennzeichnet, dass

$R_1$ Wasserstoff, Fluor oder Chlor;

$R_2$, $R_4$, $R_6$ und $R_7$ Wasserstoff;

$R_3$ Chlor oder Trifluormethoxy; und

$R_5$ Fluor oder Chlor

bedeuten.

6. Verbindung gemäss Anspruch 3 der Formel

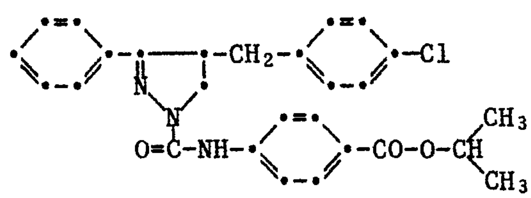

0182746

7. Verbindung gemäss Anspruch 4 der Formel

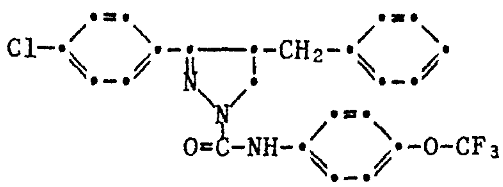

8. Verbindung gemäss Anspruch 5 der Formel

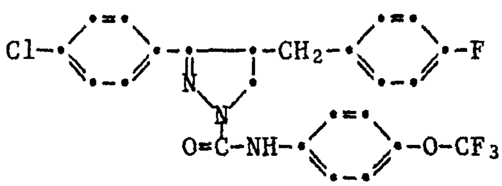

9. Verbindung gemäss Anspruch 5 der Formel

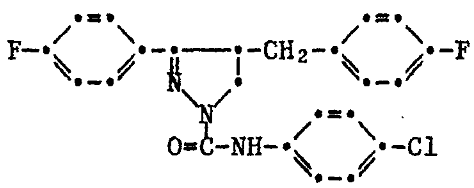

10. Verbindung gemäss Anspruch 5 der Formel

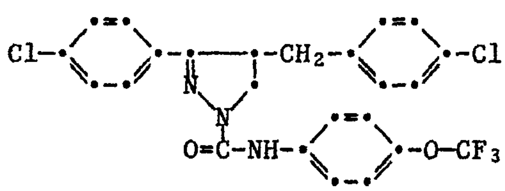

11. Verbindung gemäss Anspruch 5 der Formel

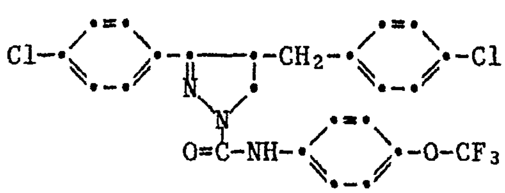

12. Verbindung gemäss Anspruch 4 der Formel

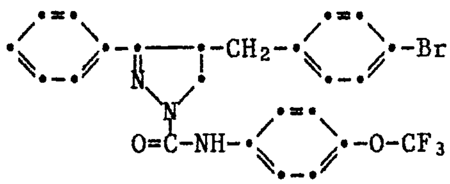

13. Verbindung gemäss Anspruch 5 der Formel

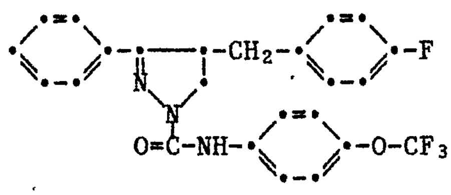

14. Verbindung gemäss Anspruch 5 der Formel

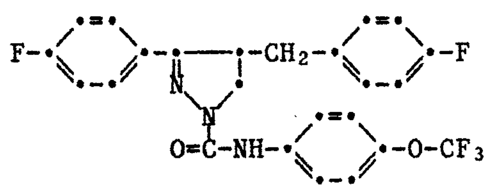

15. Verfahren zur Herstellung einer Verbindung der Formel I gemäss einem der Ansprüche 1 bis 14, dadurch gekennzeichnet, dass man eine Verbindung der Formel II

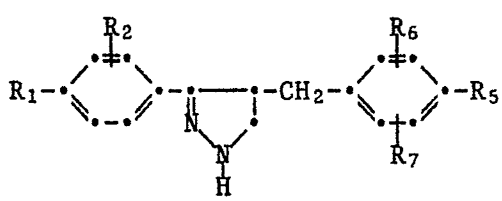

(II)

mit einer Verbindung der Formel III

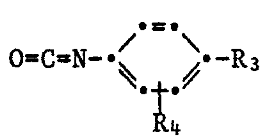

(III)

umsetzt, wobei in den Formeln II und III die Reste $R_1$ bis $R_7$ die in den Ansprüchen 1 bis 5 angegebenen Bedeutungen haben.

16. Schädlingsbekämpfungsmittel, welches als aktive Komponente eine Verbindung gemäss einem der Ansprüche 1 bis 14 zusammen mit geeigneten Trägern und/oder anderen Zuschlagstoffen enthält.

17. Verwendung einer Verbindung gemäss einem der Ansprüche 1 bis 14 zur Bekämpfung von Insekten und Vertretern der Ordnung Akarina.

18. Verwendung gemäss Anspruch 17 zur Bekämpfung larvaler Stadien pflanzenschädigender Insekten.

19. Verwendung gemäss Anspruch 17 zur Bekämpfung pflanzenschädigender Bodeninsekten.

20. Verfahren zur Bekämpfung von Insekten und Vertretern der Ordnung Akarina, dadurch gekennzeichnet, dass man die Schädlinge bzw. deren verschiedene Entwicklungsstadien oder deren Aufenthaltsort mit einer pestizid wirksamen Menge einer Verbindung der Formel I gemäss einem der Ansprüche 1 bis 14 oder mit einem Mittel enthaltend neben Zusatz- und Trägerstoffen eine pestizid wirksame Menge dieser Verbindung, in Kontakt bringt oder behandelt.

FO 7.5/EIC/cs*